# EUROPEAN PATENT SPECIFICATION

(11) **EP 1 872 717 B1**
(45) Date of publication and mention of the grant of the patent: **08.09.2010**
(21) Application number: 06396011.6
(22) Date of filing: 29.06.2006
(51) Int. Cl.: A61B 5/0424, A61B 5/00

(54) **A medical telemetry system**
Medizinisches Telemetriesystem
Système de télémétrie médicale

(43) Date of publication of application: 02.01.2008
(73) Proprietor: GENERAL ELECTRIC COMPANY, Schenectady, NY 12345 (US)
(72) Inventor: Virtanen, Juha, 00660 Helsinki (FI)
(74) Representative: Simmelvuo, Markku Kalevi

(56) References cited:
- US-A- 3 953 848
- US-A- 4 577 639
- US-A- 4 598 281
- US-A- 6 052 614
- US-A1- 2003 083 707
- US-B1- 6 267 723

## Description

### FIELD OF THE INVENTION

The invention relates to a medical telemetry system and a method for signaling leads-off information in a medical telemetry system. The medical telemetry system according to the invention comprises a sensor unit comprising ECG electrodes connected to a transmitter unit, and a receiver unit connectable to ECG input connector on a patient monitor. The ECG signal and leads-off state of the electrodes are transmitted from the sensor unit to the receiver unit using radio-wave communication. According to the present invention, leads-off information to the patient monitor is signaled by feeding a predefined low-impedance DC voltage to the ECG signal port.

### BACKGROUND OF THE INVENTION

Electrocardiography (ECG) measures the electrical activity of the heart. It depicts the rate and the regularity of heartbeat as well as the presence of cardiac diseases or damage, arrhythmias etc. ECG is measured by placing electrodes on the chest of the patient and measuring the bioelectrical potentials produced by the heart. Electrodes attached to the patient are connected by leads to an ECG monitor for further signal processing.

If there is a poor connection between the electrode and the skin of the patient, the ECG signal will become distorted by noise. Detecting the poor electrode-to-skin connection or a lead detachment is important for the medical professionals to quickly locate the faulty connection and take appropriate steps to recover the measurement. Conventional leads-off detectors use impedance measurement in determining a bad electrode-to-skin connection.

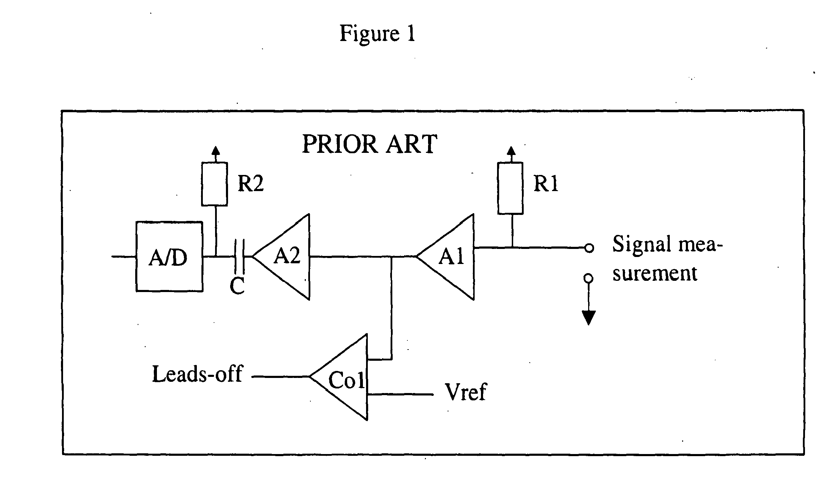

Figure 1 depicts a simplified prior art leads-off detecting circuit. The impedance of the electrodes is measured by continuously feeding a small (of the order of 10 nA) DC current through the ECG electrodes and the patient to ground through a reference electrode. The other input of the amplifier is thus connected to floating ground. If an electrode is loose or poorly connected, even such a small dc current is adequate to create a considerable difference in the voltage measured from the electrode (the magnitude of the voltage may be a couple of volts). In case of leads-off, the resistance R1 in Figure 1 forces the input of the amplifier A1 to rise to the predetermined DC-voltage of e.g. 2,5V or 5V. The voltage produced by the dc current is then compared with a reference voltage V_{ref} in comparator CO1 to determine if the impedance of the electrode is too high. When the output or the input of the second amplifier rises over the predetermined DC-voltage, it causes that the comparator switches its state and leads-off state is detected. Similarly, if the measuring electrodes are shortcircuited, the input of the amplifier goes to zero volts and the comparator CO1 switches its state. Similar leads-off detection circuit is described in publication US 4,577,639 (Simon, Mark I. et al).

Often, the monitored patient has to be moved from one location to another, for example, from the emergency rescue scene to the hospital or from one hospital unit to another. Detaching and reattaching the electrodes is time-consuming to the medical professionals and stressful to the patient, and therefore, highly undesirable. Free relative movement of the patient and the monitor is often desirable, but restricted by the measurement cable connecting the patient to the monitor. Furthermore, the typically thick and long cable between the patient and the monitor clutters easily and tends to be a nuisance for the nursing staff. In attempt to solve these problems, wireless ECGs have been developed. Publication EP 1551503 (Massicotte et al.) describes an example of a wireless ECG monitoring system. The signal acquired by electrodes attached to the patient is converted into a digital radio signal which is transmitted to a wireless receiver device. The receiver device can further be connected to a computer or a hand-held PC.

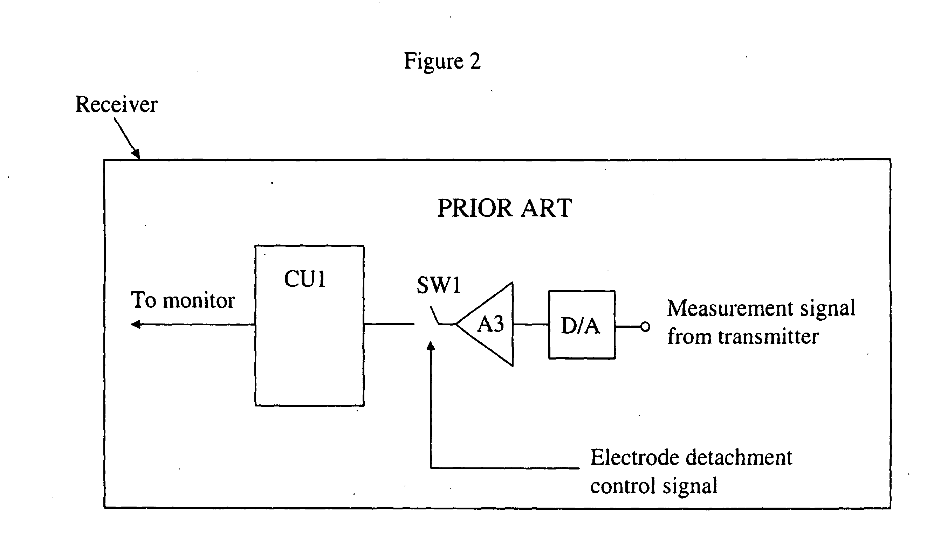

Patent publication US 6,267,723 describes a medical telemetry system in which a sensor unit detects a biomedical signal which is converted into a radio signal by a transmitter. Transmission of medical signals via radio transmission is well known as telemetry. The transmitter described in the publication has an electrode detachment detection unit for detecting electrode detachment based on the output signal of the sensor unit. The biometric signal and the signal indicative of the electrode detachment are then transmitted and received by a receiver. Simplified electrical circuitry of a similar receiver is depicted in Figure 2. The signal received from the transmitter is supplied to an amplifier A3 with a low input impedance, and further to an input of another device (the ECG monitor) through a switch SW1 and a connection unit CU1. If the electrode detachment signal is detected the switch is opened and the output impedance of the receiver's voltage output is thus changed. The switch simulates the real behavior of loose electrodes by disconnecting the measurement signal from the receiver unit and is thus an obvious and universal solution. When an ECG signal is input there is very low output impedance, but in case of lead detachment the switch is opened and the output impedance is changed to very high. The publication also makes a difference when the radio wave transmission is interrupted versus when the electrodes are detached. The system described in the publication requires complicated control logic and, a large number of components requiring significantly board space is needed to implement electrode detachment circuitry. This is expensive and may lead to unreliable or false alarms.

Patent publication US 3,953,848 discloses a system for wireless transmission of ECG measurement signals between a transmitter unit connected to ECG electrode leads and a receiver unit connected to an ECG monitor. An analogue FM-modulation is used between the transmitter and receiver units. In the transmitter unit the ECG measurements are modulated first with a subcarrier and later with a main carrier. The leads-off state is signaled from the transmitter unit to the receiver unit using a particularly low subcarrier frequency. In the actual receiver unit the leads-off state is signaled using a separate indicator and the actual ECG measurement signal is switched away from the actual output. The mentioned indicator is a separate ground that prevents the entry of the ECG signal to the ECG monitor.

Patent publication US 6,052,614 discloses a system for transmitting ECG measurement signals on an optical fiber. The system has been designed with MRI in mind for situations where the ECG monitoring must be performed during MRI. In the system of US 6,052,614 short high-resistance leads are connected to the ECG electrodes. The leads are connected via an RF-filter to an optical transmitter fitted in a Faraday cage. The optical transmitter is connected to an optical receiver with the optical fiber. The ECG monitor is connected to the optical receiver. If the transmitter detects a leads-off state, the ECG signal voltage is raised be-yond the normal voltage interval, for example, by feeding a DC voltage to the ECG lead selection unit of the transmitter unit, in addition to the ECG signal fed. The driving of the ECG signal out of range is done by a circuit in the lead select network which resides in the ECG electronics sensor module. The ECG electronics sensor module operates the transmitter and is located in the transmitter side.

Further problem in prior art systems is that A/D conversion of an ECG signal with a large offset voltage is not practical or possible to arrange without radically decreasing the resolution of the conversion. It is thus necessary to perform detecting of the leads-off state before digital transmission of the signal to the ECG monitor.

### PURPOSE OF THE INVENTION

The purpose of the invention is to provide a simple and reliable method and system for signaling leads-off information in a medical telemetry system.

### SUMMARY OF THE INVENTION

The invention relates to a medical telemetry system according to claim 1 and a method according to claims 12 for signaling leads-off information in a medical telemetry system.

The output impedance of the voltage source is low. Typically it is in the range between 1 and 100 ohms. In any case it should be less than 100E6 ohms.

In one embodiment of the invention the output impedance of the receiver unit is arranged to remain constant regardless of the leads-off information. The output impedance of the amplifier unit in the receiver does thus not change when changing over from normal operation to leads-off state and vice versa.

In another embodiment of the invention the receiver unit is further arranged to comprise means for detecting radio wave disconnection state and/or means for signaling the radio wave disconnection state information to the patient monitor by feeding a predefined DC voltage to the ECG signal port.

In yet another embodiment of the invention the output impedance of the receiver unit is less than 100E6 ohms.

The medical telemetry system according to the invention can further comprise an indicator for indicating the leads-off state to a user of the system. The receiver unit of the medical telemetry system according to the invention can further be arranged to comprise an indicator such as a LED for indicating the radio wave disconnection state to a user of the medical telemetry system.

In addition, the leads-off information and radio wave disconnection information can both independently activate the feeding of the DC-voltage to the ECG signal port.

In one embodiment of the invention, the transmitter unit comprises an impedance measurement circuit for detecting the leads-off state.

The transmitter unit and the receiver unit can further be paired so as to be interchangeably connected to each other.

The transmitter unit of the present invention can further comprise means for detecting a pacemaker in the said ECG signal. In order to transmit the pacemaker peak timing information to the monitor, sharp voltage spikes are superimposed on the reconstructed ECG signal.

The benefits of the invention are related to the improved reliability of leads-off measurement and signaling in a medical telemetry system. Voltage mode signaling is more reliable than high impedance signaling. In addition, feeding a predefined low-impedance DC voltage to the ECG signal port requires only relatively simple electronic circuitry and is therefore, also relatively inexpensive to manufacture. A further benefit of the invention is that it allows the medical professionals to move the patient safely and easily while providing the possibility to change a new monitor to receive the ECG and leads-off signals from the sensor unit. It also relieves the stress experienced by the patient and artifacts in the ECG signal caused by the stress because there is no need to disconnect and reapply the measuring electrodes to the chest of the patient as the sensor unit (the electrodes and the transmitter unit) of the present invention may stay in place even if the patient is moved from one location to another.

Various other features, objects, and advantages of the invention will further be apparent from the following detailed description and the drawings.

### BRIEF DESCRIPTION OF THE DRAWINGS

The accompanying drawings, which are included to provide a further understanding of the invention and constitute a part of this specification, illustrate embodiments of the invention and together with the description help to explain the principles of the invention. In the drawings:
Fig. 1 is schematic presentation of a simplified prior art leads-off detecting circuit;
Fig. 2 depicts a simplified electrical circuitry of a receiver unit of a medical telemetry system, in which the output impedance is changed in response to a detected leads-off state;
Fig. 3 depicts the ECG measurement system according to the present invention; and
Fig. 4 is a simplified presentation of the electronics circuits of the transmitter and receiver units according to the present invention.

### DETAILED DESCRIPTION OF THE EMBODIMENTS

Reference will now be made in detail to the embodiments of the present invention, examples of which are illustrated in the accompanying drawings.

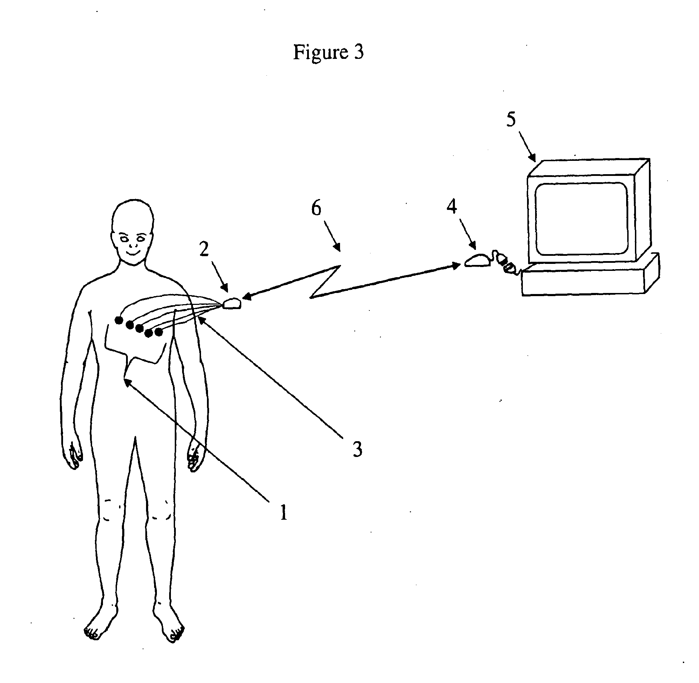

The ECG measurement system according to the present invention is illustrated in Figure 3. The sensing unit comprises electrodes 1 connected to the patient for detecting biometric voltages and further connected to a transmitter unit 2 by leads 3 for transmitting the detected signals. Transmitter unit 2 contains, for example, a high gain amplifier, an analog to digital (A/D) converter and an antenna. For power acquisition, the transmitter may use a chargeable battery. The transmitter 2 performs the impedance measurement of the electrodes and detects when there is a poor electrode-to-skin connection or a lead detachment situation i.e. *leads-off state of the electrodes* occurs. When one or more of the electrodes is poorly attached on the skin, the transmitter unit 2 outputs a leads-off state signal and transmits it to the receiver unit using radio link 6. The transmitted signal is received by the receiver unit 4. The transmitted signal is sent to the ECG signal port of the patient monitor for further signal processing. The receiver unit 4 also contains an amplifier and an antenna. In addition, it contains a digital to analog (D/A) converter. The receiver unit may further comprise an indicator such as a LED for indicating the leads-off state to the user of the system. The ECG monitoring system also detects if the patient has a pacemaker. If a pacemaker is detected, time stamps are regularly added to the transmitted ECG signal and in the receiver unit a narrow peak is added to the signal, which looks like a pacemaker to the monitor.

When the receiver unit receives the leads-off state information, it signals the leads-off state to the monitor by driving a predefined low-impedance DC-voltage to the output pins of the receiver unit. The signaling unit signaling the leads-off state to the monitor thus comprises at least the DC voltage source. This voltage is of the order of 2 V, whereas the ECG signal under normal conditions is significantly smaller (usually about 1 - 10 mV). When the leads-off state disappears, the DC voltage signal for the monitor is removed.

The transmitter and receiver units can be paired to operate together interchangeably. This can be implemented, for example, by push-buttons in the receiver/transmitter units that are pressed simultaneously to establish the initial set-up communication between the transmitter and the receiver units. Pressing buttons simultaneously may temporarily interrupt the measurement function in the transmitter unit. Initial set-up to accomplish pairing is done every time when a new patient is connected to the monitor. If the patient is transferred from one care area to another, it is convenient to keep the measurement electrodes at place and the sensor unit (the electrodes and the transmitter) active. When the patient enters a new care area with the sensor unit, there is another monitor with receiver unit waiting. After the set-up communication of the receiver and transmitter units, the receiver will accept only information from the corresponding transmitter and ignore other transmissions if there is other equipment functioning in the same frequency band.

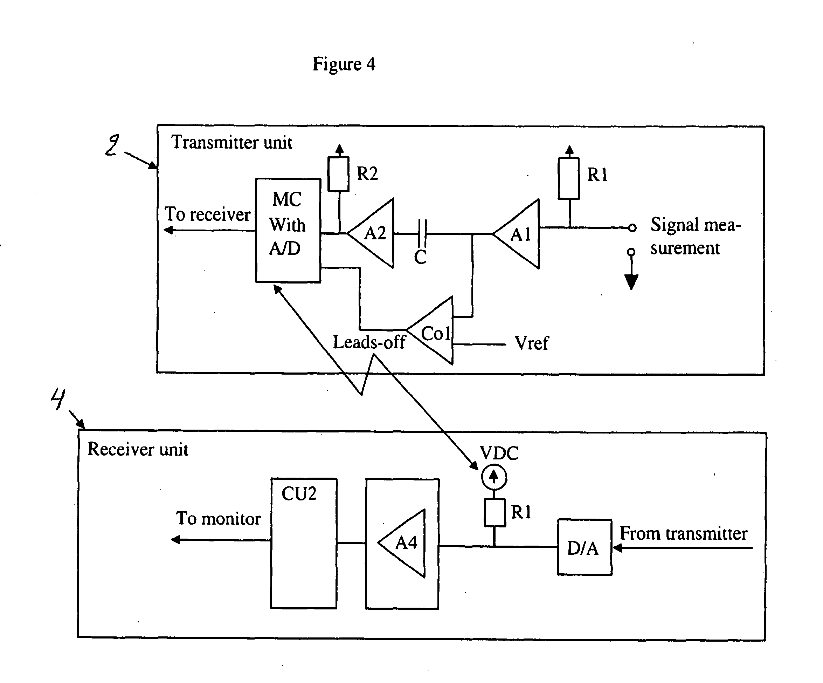

Figure 4 illustrates the simplified structure of the transmitter and receiver units according to the present invention. The transmitter unit 2 performs the impedance measurement of the electrodes and in case of detecting a leads-off state, transmits leads-off information to the receiver unit 4. The impedance measurement is implemented in the transmitter similarly to prior art measurement described in the section referring to Figure 1. The transmitter further includes a microcontroller MC with A/D converter and other circuitry needed for implementing digital transmission protocols such as the Bluetooth^{™}. When the receiver 8 receives the leads-off signal from the transmitter, the voltage level at the input side of the amplifier unit A4 is altered by feeding a predefined low-impedance DC voltage V_{DC} to the amplifier unit A4. The low-impedance output stage of the amplifier unit A4 is connected directly to the connector unit CU2. The "leads-off information" thus changes the output voltage of the D/A conversion unit, which output voltage is transferred by the amplifier unit A4 to connection unit CU2. The output impedance of the amplifier unit determines also the impedance seen from the connection unit. In the system according to the present invention, the output impedance of the amplifier unit A4 is constantly low regardless of the level of the output voltage. Because of the prior art design of the impedance measurement, the high DC voltage has same logical effect for the amplifier as high output impedance. This means that the monitor processes the high DC voltage in the amplifier output similarly to high output impedance of the amplifier unit A4. Generating a high DC voltage is technically advantageous over high output impedance, because it leads to less complex electronics circuit in the receiver unit.

In addition to a leads-off state, ECG signal acquisition can be disconnected if the radio wave communication between the transmission unit and the receiver unit is interrupted. If the receiver is unable to detect a signal *a radio wave disconnection state* is activated in the receiver unit. The receiver unit indicates the *radio wave disconnection state* for the user using a visual indicator (for example, a LED) on the receiver unit. This indicator is different from the indicator for the *leads-off state of the elec*trodes although there may also be another indicator for the leads-off state in the receiver. The receiver unit signals the *radio wave disconnection state* to the monitor by driving a predefined DC voltage to its output pins. Therefore, irrespective of which state (radio wave disconnection state or leads-off state) receiver unit detects, its function is the same and a DC voltage is output from the receiver unit to the monitor. Consequently, on the monitor display "leads-off" message is displayed for both disconnections and additional information about the state of the radio link and/or the leads-off state can be obtained from visual indicators on the receiver unit.

Inside the receiver unit, these two states are exclusive. Information on the *leads-off state of the electrodes* comes from the sensor unit via radiofrequency RF link. If the RF communication is interrupted, *radio wave disconnection state* is activated and *leads-off state* is deactivated, because no such information is available. To the monitor port these both states generate an identical message in the form of a high DC voltage, but when *radio wave disconnection state* is activated there is no more real-time or memory-stored information about the possible *leads-off state* in the receiver or in the monitor. Hence, in such a transition state, where *leads-off state* is active at the moment when *radio wave disconnection state* is activated, the high DC voltage is maintained without interruption. The user may first check and correct the radio link and then determine whether the *leads-off state* is still activated.

It must be contemplated that the above embodiments of the invention are presented here as examples and that the basic idea of the invention may vary within the scope of the claims. It will also be evident to a person skilled in the art that with the advancement of technology, the idea of the invention may be implemented in various other ways. The invention and its embodiments are thus not limited to the examples described above; instead they may vary within the scope of the claims.

## Claims

1. A medical telemetry system, the system comprising:
a patient monitor (5) comprising an ECG input connector and an ECG signal port;
a sensor unit in proximity with the patient further comprising a transmitter unit (2) and ECG electrodes (1) to be attached on a patient and to be connected to the transmitter unit (2)
a receiver unit (4) connected to the ECG input connector of the patient monitor (5), and configured to receive an ECG signal and leads-off state information via wireless radio-wave communication from the transmitter unit (2), and to feed a reconstructed ECG signal to the ECG input connector of the patient monitor (5), **characterized in that** the receiver unit (4) further comprises:
means comprising at least a voltage source controlled by the leads-off state information for signaling the leads-off information to the patient monitor (5) by feeding a predefined DC voltage greater than 1V to the ECG signal port.

2. The medical telemetry system according to claims 1 or 2, **characterized in that** the output impedance of the receiver unit (4) is configured to remain constant when changing over from normal operation to the leads-off state.

3. The medical telemetry system according to claims 1 or 2, **characterized in that** said receiver unit (4) is further configured to detect radio wave disconnection state.

4. The medical telemetry system according to any one of the preceding claims 1 - 3, **characterized in that** the output impedance of the receiver unit (4) is less than 100E6 ohms.

5. The medical telemetry system according to any one of the preceding claims 1 - 4, **characterized in that** said transmitter unit (2) comprises an impedance measurement circuit for detecting the leads-off state.

6. The medical telemetry system according to any of the preceding claims 1 - 5, **characterized in that** said system further comprises an indicator configured to indicate the leads-off state to a user of the system.

7. The medical telemetry system according to claim 3 **characterized in that** said receiver unit (4) further comprises:
an indicator configured to indicate the radio wave disconnection state to a user of the medical telemetry system.

8. The medical telemetry system according to any one of the preceding claims 1 - 7, **characterized in that** said transmitter unit (2) and said receiver unit (4) are arranged to be paired so as to be interchangeably connected to each other.

9. The medical telemetry system according to any one of the preceding claims 1 - 8, **characterized in that** said transmitter unit (2) is further configured to detect a pacemaker in the ECG signal.

10. The medical telemetry system according to any one of the preceding claims 1 - 9, **characterized in that** the receiver unit (4) is arranged to superimpose sharp voltage spikes on the reconstructed ECG signal in order to transmit a pacemaker peak timing information to the patient monitor (5).

11. The medical telemetry system according to claims 3 or 7, **characterized in that** the receiver unit (4) is further arranged to activate the feeding of the DC voltage to the ECG signal port upon detection of the radio wave disconnection state.

12. A method for signaling leads-off information in a medical telemetry system, the method comprising:
arranging a sensor unit in proximity with a patient, the sensor unit comprising a transmitter unit (2) and ECG electrodes (1) to be attached on the patient and to be connected to the transmitter unit (2);
receiving by a receiver unit (4) connected to an ECG input connector of a patient monitor (5) comprising an ECG signal port, an ECG signal and leads-off state information via wireless radio-wave communication from the transmitter unit (2); and
feeding by said receiver unit (4) a reconstructed ECG signal to the ECG input connector of the patient monitor, **characterized in that** the method further comprises:
signaling the leads-off state information to the patient monitor (5) by feeding a predefined DC voltage greater than 1V from at least a voltage source controlled by the leads-off state information in the receiver unit (4) to the ECG signal port.

13. The method for signaling leads-off information in a medical telemetry system according to claim 12, **characterized in that** the method further comprises the step:
arranging the output impedance of the receiver unit (4) to remain constant when changing over from normal operation to leads-off state.

14. The method for signaling leads-off information in a medical telemetry system according to claims 12 or 13, **characterized in that** the method further comprises the step:
detecting a radio wave disconnection state by said receiver unit (4).

15. The method according to anyone of the preceding claims 12 - 14, **characterized in that** the method further comprises the step:
adjusting the output impedance of the receiver unit (4) to less than 100E6 ohms.

16. The method according to any one of the preceding claims 12 - 15, **characterized in that** the method further comprises:
detecting the leads-off state by an impedance measurement circuit in said transmitter unit (2).

17. The method according to any one of the preceding claims 12 - 16, **characterized in that** the method further comprises:
indicating by an indicator of said system the leads-off state to a user of the system.

18. The method according to claim 14, **characterized in that** the method further comprises:
indicating by an indicator of said receiver unit (4) the radio wave disconnection state to a user of the medical telemetry system.

19. The method according to any one of the preceding claims 12 - 18, **characterized in that** the method further comprises:
pairing said transmitter unit (2) and said receiver unit (4) so as to be interchangeably connected to each other.

20. The method according to any one of the preceding claims 12 - 19, **characterized in that** the method further comprises:
detecting a pacemaker from the ECG signal by said transmitter unit (2).

21. The method according to any one of the preceding claims 12 - 20, **characterized in that** the method further comprises:
superimposing sharp voltage spikes on the reconstructed ECG signal in order to transmit a pacemaker peak timing information to the patient monitor (5).

22. The method according to claims 14 or 18, **characterized in that** the method further comprises:
activating the feeding of the predefined DC voltage to the ECG signal port by the detected radio wave disconnection state.

## Patentansprüche

1. Medizinisches Telemetriesystem, wobei das System folgendes aufweist:
- einen Patientenmonitor, der einen ECG-Eingangsverbinder und einen ECG-Signalanschluss aufweist;
- eine Sensoreinheit in der Nähe des Patienten, die ferner eine Sendereinheit (2) sowie ECG-Elektroden (1) aufweist, die an einem Patienten anzubringen sind und die mit der Sendereinheit (2) zu verbinden sind;
- eine Empfängereinheit (4), die mit dem ECG-Eingangsverbinder des Patientenmonitors (5) verbunden und konfiguriert ist, um ein ECG-Signal sowie Informationen über nicht angeschlossene Leitungen über eine drahtlose Funkwellenverbindung von der Sendereinheit (2) zu empfangen und dem ECG-Eingangsverbinder des Patientenmonitors (5) ein rekonstruiertes ECG-Signal zu liefern,
**dadurch gekennzeichnet,**
**dass** die Empfängereinheit ferner folgendes aufweist:
eine Einrichtung, die zumindest eine Spannungsquelle aufweist, welche von den Informationen über nicht angeschlossene Leitungen gesteuert wird, um die Informationen über nicht angeschlossene Leitungen als Signal an den Patientenmonitor (5) abzugeben, indem eine vorher definierte Gleichspannung von mehr als 1 V dem ECG-Signalanschluss zugeführt wird.

2. Medizinisches Telemetriesystem nach Anspruch 1,
**dadurch gekennzeichnet,**
**dass** die Ausgangsimpedanz der Empfängereinheit (4) so konfiguriert ist, dass sie konstant bleibt, wenn ein Übergang aus dem Normalbetrieb in den Zustand mit nicht angeschlossenen Leitungen stattfindet.

3. Medizinisches Telemetriesystem nach Anspruch 1 oder 2,
**dadurch gekennzeichnet,**
**dass** die Empfängereinheit (4) ferner derart konfiguriert ist, dass sie einen Funkwellen-Unterbrechungszustand detektiert.

4. Medizinisches Telemetriesystem nach einem der Ansprüche 1 bis 3,
**dadurch gekennzeichnet,**
**dass** die Ausgangsimpedanz der Empfängereinheit (4) geringer als 100 E6 Ohm ist.

5. Medizinisches Telemetriesystem nach einem der Ansprüche 1 bis 4,
**dadurch gekennzeichnet,**
**dass** die Sendereinheit eine Impedanz-Messschaltung aufweist, um den Zustand von nicht angeschlossenen Leitungen zu detektieren.

6. Medizinisches Telemetriesystem nach einem der Ansprüche 1 bis 5,
**dadurch gekennzeichnet,**
**dass** das System ferner eine Anzeige aufweist, die derart konfiguriert ist, dass sie einem Benutzer des Systems den Zustand von nicht angeschlossenen Leitungen anzeigt.

7. Medizinisches Telemetriesystem nach Anspruch 3,
**dadurch gekennzeichnet,**
**dass** die Empfängereinheit (4) ferner folgendes aufweist:
eine Anzeige, die derart konfiguriert ist, dass sie den Funkwellen-Unterbrechungszustand einem Benutzer des medizinischen Telemetriesystems anzeigt.

8. Medizinisches Telemetriesystem nach einem der Ansprüche 1 bis 7,
**dadurch gekennzeichnet,**
**dass** die Sendereinheit (2) und die Empfängereinheit (4) paarweise derart angeordnet sind, dass sie austauschbar miteinander verbunden sind.

9. Medizinisches Telemetriesystem nach einem der Ansprüche 1 bis 8,
**dadurch gekennzeichnet,**
**dass** die Sendereinheit (2) ferner derart konfiguriert ist, dass sie einen Schrittmacher in dem ECG-Signal detektiert.

10. Medizinisches Telemetriesystem nach einem der Ansprüche 1 bis 9,
**dadurch gekennzeichnet,**
**dass** die Empfängereinheit (4) derart angeordnet ist, dass sie scharfe Spannungsspitzen dem rekonstruierten ECG-Signal überlagert, um dem Patientenmonitor (5) Spitzenzeitinformationen des Schrittmachers zu senden.

11. Medizinisches Telemetriesystem nach Anspruch 3 oder 7,
**dadurch gekennzeichnet,**
**dass** die Empfängereinheit (4) ferner derart angeordnet ist, dass sie die Zuführung des Gleichspannungssignals zu dem ECG-Signalanschluss beim Detektieren des Funkwellen-Unterbrechungszustandes aktiviert.

12. Verfahren zur Signalabgabe für Informationen über nicht angeschlossene Leitungen in einem medizinischen Telemetriesystem,
wobei das Verfahren folgendes aufweist:
- Anordnen einer Sensoreinheit in der Nähe von einem Patienten, wobei die Sensoreinheit eine Sendereinheit (2) und ECG-Elektroden (1) aufweisen, die an dem Patienten anzubringen sind und die mit der Sendereinheit (2) zu verbinden sind;
- Empfangen mittels einer Empfängereinheit (4), die an einen ECG-Signalverbinder eines Patientenmonitors (5) angeschlossen ist, der einen ECG-Signalanschluss aufweist, von einem ECG-Signal und Informationen über nicht angeschlossene Leitungen über eine drahtlose Funkwellen-Kommunikation von der Sendereinheit (2);
und
- Zuführen mittels der Empfängereinheit (4) von einem rekonstruierten ECG-Signal an den ECG-Eingangsverbinder des Patientenmonitors,
**dadurch gekennzeichnet,**
**dass** das Verfahren ferner folgendes aufweist:
Signalabgabe von Informationen über nicht angeschlossene Leitungen zu dem Patientenmonitor (5) durch Zuführen einer vorher definierten Gleichspannung, die größer als 1 V ist, von zumindest einer Spannungsquelle, die in der Empfängereinheit (4) von den Informationen über nicht angeschlossene Leitungen gesteuert wird, an den ECG-Signalanschluss.

13. Verfahren zur Signalabgabe für Informationen über nicht angeschlossene Leitungen in einem medizinischen Telemetriesystem gemäß Anspruch 12,
**dadurch gekennzeichnet,**
**dass** das Verfahren ferner folgenden Schritt aufweist:
Einstellen, dass die Ausgangsimpedanz der Empfängereinheit konstant bleibt, wenn ein Übergang aus dem Normalbetrieb in den Zustand mit nicht angeschlossenen Leitungen stattfindet.

14. Verfahren zur Signalabgabe für Informationen über nicht angeschlossene Leitungen in einem medizinischen Telemetriesystem gemäß Anspruch 12 oder 13,
**dadurch gekennzeichnet,**
**dass** das Verfahren ferner folgenden Schritt aufweist:
Detektieren eines Funkwellen-Unterbrechungszustandes mittels der Empfängereinheit (4).

15. Verfahren nach einem der vorhergehenden Ansprüche 12 bis 14,
**dadurch gekennzeichnet,**
**dass** das Verfahren ferner folgenden Schritt aufweist:
Einstellen der Ausgangsimpedanz der Empfängereinheit (4) auf einen Wert von weniger als 100 E6 Ohm.

16. Verfahren nach einem der vorhergehenden Ansprüche 12 bis 15,
**dadurch gekennzeichnet,**
**dass** das Verfahren ferner folgendes aufweist:
Detektieren des Zustands von nicht angeschlossenen Leitungen mit einer Impedanz-Messschaltung in der Sendereinheit (2).

17. Verfahren nach einem der vorhergehenden Ansprüche 12 bis 16,
**dadurch gekennzeichnet,**
**dass** das Verfahren ferner folgendes aufweist:
Anzeigen des Zustands von nicht angeschlossenen Leitungen mittels einer Anzeige für einen Benutzer des Systems.

18. Verfahren nach Anspruch 14,
**dadurch gekennzeichnet,**
**dass** das Verfahren ferner folgendes aufweist:
Anzeigen des Funkwellen-Unterbrechungszustandes mittels einer Anzeige der Empfängereinheit (4) für einen Benutzer des medizinischen Telemetriesystems.

19. Verfahren nach einem der vorhergehenden Ansprüche 12 bis 18,
**dadurch gekennzeichnet,**
**dass** das Verfahren ferner folgendes aufweist:
paarweises Anordnen von der Sendereinheit (2) und der Empfängereinheit (4), so dass diese austauschbar miteinander verbunden sind.

20. Verfahren nach einem der vorhergehenden Ansprüche 12 bis 19,
**dadurch gekennzeichnet,**
**dass** das Verfahren ferner folgendes aufweist:
Detektieren eines Schrittmachers aus dem ECG-Signal von der Sendereinheit (2).

21. Verfahren nach einem der vorhergehenden Ansprüche 12 bis 20,
**dadurch gekennzeichnet,**
**dass** das Verfahren ferner folgendes aufweist:
Überlagern von scharfen Spannungsspitzen auf dem rekonstruierten ECG-Signal, um dem Patientenmonitor (5) eine Spitzenzeitinformation des Schrittmachers zu senden.

22. Verfahren nach Anspruch 14 oder 18,
**dadurch gekennzeichnet,**
**dass** das Verfahren ferner folgendes aufweist:
Aktivieren der Zuführung der vorher definierten Gleichspannung zu dem ECG-Signalanschluss durch den detektierten Funkwellen-Unterbrechungszustand.

## Revendications

1. Système de télémétrie médicale, le système comprenant :
un moniteur de patient (5), comprenant un connecteur d'entrée ECG et un port de signal ECG ;
une unité de capteur, à proximité du patient, comprenant en outre une unité émettrice (2) et des électrodes ECG (1), devant être attachées sur un patient et devant être connectées à l'unité émettrice (2) ;
une unité réceptrice (4), connectée au connecteur d'entrée ECG du moniteur de patient (5) et configurée pour recevoir un signal ECG et une information d'état de déconnexion, via une communication sans fil par ondes radio à partir de l'unité émettrice (2), et pour fournir un signal ECG reconstruit au connecteur d'entrée ECG du moniteur de patient (5), **caractérisé en ce que** l'unité réceptrice (4) comprend en outre :
des moyens, comprenant au moins une source de tension, commandée par l'information d'état de déconnexion, pour signaler l'information d'état de déconnexion au moniteur de patient (5), par fourniture d'une tension de courant continu CC prédéfinie, supérieure à 1 V, au port de signal ECG.

2. Système de télémétrie médicale selon la revendication 1 ou 2, **caractérisé en ce que** l'impédance de sortie de l'unité réceptrice (4) est configurée pour rester constante lors du passage du fonctionnement normal à l'état de déconnexion.

3. Système de télémétrie médicale selon la revendication 1 ou 2, **caractérisé en ce que** ladite unité réceptrice (4) est en outre configurée pour détecter un état de déconnexion vis-à-vis des ondes radio.

4. Système de télémétrie médicale selon l'une quelconque des revendications 1 à 3, **caractérisé en ce que** l'impédance de sortie de l'unité réceptrice (4) est inférieure à 100E6 ohms.

5. Système de télémétrie médicale selon l'une quelconque des revendications 1 à 4, **caractérisé en ce que** ladite unité émettrice (2) comprend un circuit de mesure d'impédance, pour détecter l'état de déconnexion.

6. Système de télémétrie médicale selon l'une quelconque des revendications 1 à 5, **caractérisé en ce que** ledit système comprend en outre un indicateur, configuré pour indiquer l'état de déconnexion à un utilisateur du système.

7. Système de télémétrie médicale la revendication 3, **caractérisé en ce que** ladite unité réceptrice (4) comprend en outre :
un indicateur, configuré pour indiquer l'état de déconnexion vis-à-vis des ondes radio à un utilisateur du système de télémétrie médicale.

8. Système de télémétrie médicale selon l'une quelconque des revendications 1 à 7, **caractérisé en ce que** ladite unité émettrice (2) et ladite unité réceptrice (4) sont agencés pour être appairées de manière à être connectées l'une à l'autre de manière interchangeable.

9. Système de télémétrie médicale selon l'une quelconque des revendications 1 à 8, **caractérisé en ce que** ladite unité émettrice (2) est en outre configurée pour détecter un stimulateur cardiaque dans ledit signal ECG.

10. Système de télémétrie médicale selon l'une quelconque des revendications 1 à 9, **caractérisé en ce que** l'unité réceptrice (4) est agencée pour superposer des pics de tension aigus au signal ECG reconstruit, de manière à transmettre une information de positionnement temporel de crête de stimulateur cardiaque au moniteur de patient (5).

11. Système de télémétrie médicale selon la revendication 3 ou 7, **caractérisé en ce que** l'unité réceptrice (4) est en outre agencée pour activer l'alimentation de la tension de courant continu CC au port de signal ECG, lors de la détection de déconnexion vis-à-vis des ondes radio.

12. Procédé de signalisation d'une information de déconnexion dans un système de télémétrie médicale, le procédé comprenant :
l'agencement d'une unité de capteur, à proximité d'un patient, l'unité de capteur comprenant une unité émettrice (2) et des électrodes ECG (1), devant être attachées sur le patient et devant être connectées à l'unité émettrice (2) ;
la réception, par une unité réceptrice (4) connectée à un connecteur d'entrée ECG d'un moniteur de patient (5) comprenant un port de signal ECG, d'un signal ECG et d'une information d'état de déconnexion, via une communication sans fil par ondes radio à partir de l'unité émettrice (2), et
la fourniture, par ladite unité réceptrice (4), d'un signal ECG reconstruit au connecteur d'entrée ECG du moniteur de patient, **caractérisé en ce que** le procédé comprend en outre :
la signalisation de l'information d'état de déconnexion au moniteur de patient (5), par fourniture au port de signal ECG d'une tension de courant continu CC prédéfinie, supérieure à 1 V, à partir d'au moins une source de tension commandée par l'information d'état de déconnexion dans l'unité réceptrice (4).

13. Procédé de signalisation d'une information de déconnexion dans un système de télémétrie médicale selon la revendication 12, **caractérisé en ce que** le procédé comprend en outre l'étape :
d'agencement de l'impédance de sortie de unité réceptrice (4) pour rester constante lors du passage du fonctionnement normal à l'état de déconnexion.

14. Procédé de signalisation d'une information de déconnexion dans un système de télémétrie médicale selon la revendication 12 ou 13, **caractérisé en ce que** le procédé comprend en outre l'étape de :
détection d'un état de déconnexion vis-à-vis des ondes radio par ladite unité réceptrice (4).

15. Procédé selon l'une quelconque des revendications 12 à 14, **caractérisé en ce que** le procédé comprend en outre l'étape :
d'ajustement de l'impédance de sortie de l'unité réceptrice (4), pour qu'elle soit inférieure à 100E6 ohms.

16. Procédé de signalisation selon l'une quelconque des revendications 12 à 15, **caractérisé en ce que** le procédé comprend en outre :
la détection de l'état de déconnexion par un circuit de mesure d'impédance, dans ladite unité émettrice (2).

17. Procède selon l'une quelconque des revendications 12 à 16, **caractérisé en ce que** le procédé comprend en outre :
l'indication, par un indicateur dudit système, de l'état de déconnexion à un utilisateur du système.

18. Procédé selon la revendication 14, **caractérisé en ce que** le procédé comprend en outre :
l'indication, par un indicateur de ladite unité réceptrice (4), de l'état de déconnexion vis-à-vis des ondes radio à un utilisateur du système de télémétrie médicale.

19. Procède selon l'une quelconque des revendications 12 à 18, **caractérisé en ce que** le procédé comprend en outre :
l'appairage de ladite unité émettrice (2) et de ladite unité réceptrice (4), de manière à être connectées l'une à l'autre de manière interchangeable.

20. Procédé selon l'une quelconque des revendications 12 à 19, **caractérisé en ce que** le procédé comprend en outre :
la détection d'un stimulateur cardiaque dans le signal ECG, par ladite unité émettrice (2).

21. Procède selon l'une quelconque des revendications 12 à 20, **caractérisé en ce que** le procédé comprend en outre :
la superposition de pics de tension aigus au signal ECG reconstruit, de manière à transmettre une information de positionnement temporel de crête de stimulateur cardiaque au moniteur de patient (5).

22. Procédé selon la revendication 14 ou 18, **caractérisé en ce que** le procédé comprend en outre :
l'activation de l'alimentation de la tension CC prédéfinie au port de signal ECG, par la détection de l'état de déconnexion vis-à-vis des ondes radio.
